# EUROPEAN PATENT APPLICATION

(11) **EP 1 529 487 A1**
(43) Date of publication of application: **11.05.2005**
(21) Application number: 03741205.3
(22) Date of filing: 04.07.2003
(51) Int. Cl.: A61B 5/042

(54) **ELECTROCARDIOGRAM ANALYSIS DEVICE AND METHOD THEREOF**

(30) Priority: 04.07.2002 JP 2002195567
(71) Applicant: Dainippon Pharmaceutical Co., Ltd., Osaka-shi, Osaka 541-8524 (JP)
(72) Inventor: NAGATA, Shinya, Kobe-shi, Hyogo 658-0062 (JP); NAGAI, Ryuji, Takatsuki-shi, Osaka 569-1024 (JP); KOUCHI, Kenji, Toyonaka-shi, Osaka 560-0002 (JP)
(74) Representative: Arth, Hans-Lothar, Dr.
(86) International application number: PCT/JP2003/008553
(87) International publication number: WO 2004/004561

(57) **Abstract**

An electrocardiogram (ECG) analysis device and method capable of easily aiding in the diagnosis of heart disease. A CPU of ECG radar chart device runs 12-lead ECG on a patient. The CPU extracts identified values (which are based on P wave, Q wave, R wave, S wave, ST segment, or T wave) from the ECG waveform. The CPU executes the process that determines identified value level for each identified value and the process that determines the Minnesota code by utilizing information including the determined identified values. The CPU displays the determined identified value level and the Minnesota code. The CPU returns to the first step and repeats the process that generates the ECG radar chart for next heartbeat.

## Description

### CROSS-REFERENCE TO RELATED APPLICATIONS

The present application claims the benefit of patent application number 2002-195567, filed in Japan on July 4, 2002, and the subject matter of which is hereby incorporated herein by reference.

### BACKGROUND OF THE INVENTION

### 1. Field of the Invention

The present invention relates to a device and a method for analyzing an electrocardiogram and more particularly for aiding in the diagnosis of heart disease based on electrocardiogram interpretation.

### 2. Description of the Related Art

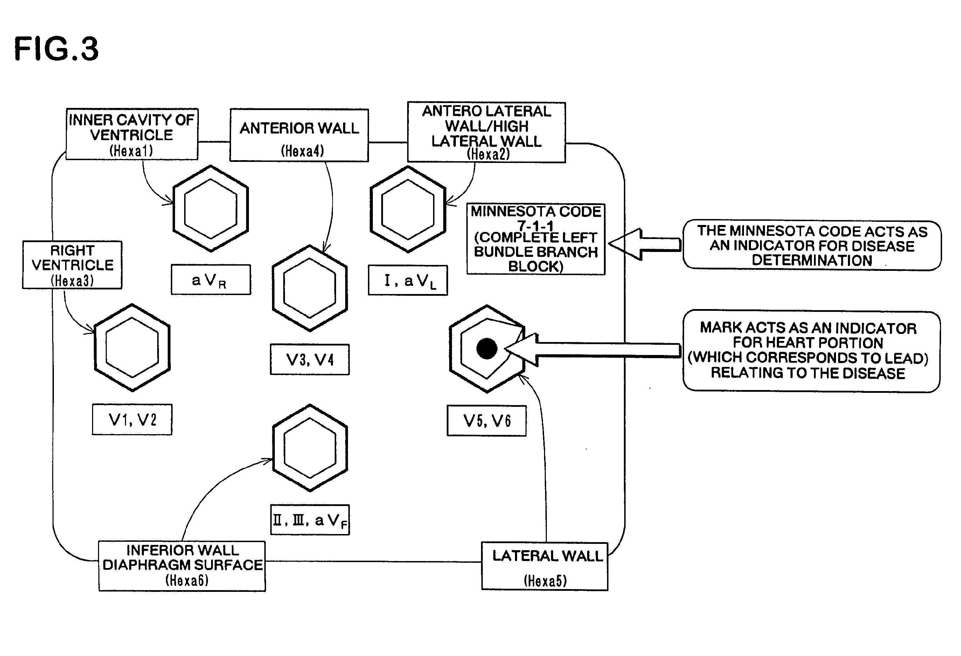

Several techniques that facilitate the analysis of disease information assessed by electrocardiogram ("ECG" as an abbreviation) have been developed. An example of such a technique includes the bar graph display technique for displaying movement of the feature points of an ECG in a bar graph form. More specifically, Japanese Patent Laid-Open No. Hei 6-181899 discloses a display technique for displaying movement of the ST level on a 12-lead ECG in bar graph form (refer to Fig. 3 etc.). By "12-lead ECG" is meant 12 ECG measurements made by attaching from several to a dozen or so electrodes to a living body.

In addition, the Minnesota code has been utilized as an ECG automatic analysis program. The Minnesota code is one of the ECG classification reference. The Minnesota code has been developed to improve objectivity and uniformity of the ECG classification and to minimize analysis errors. The Minnesota code includes the ECG classification of disease severity. In the ECG automatic analysis program utilizing the Minnesota code, generally, the classified code name is outputted.

The above-mentioned technologies, which include utilizing only specific feature points or outputting the code name base on the Minnesota code classification, are capable of facilitating the ECG analysis and aiding in the diagnosis of heart disease.

However, in medical activities, more advanced technologies that can help in discrimination from among greater varieties of heart disease are required. For example, in emergency medical activities, it is preferable to determine appropriate hospitals or services promptly to which the patients should be transferred under an aid of more comprehensive and in-depth diagnosis of heart disease.

### SUMMARY OF THE INVENTION

In one aspect, the invention provides a device and a method that are capable of providing aid to ease the means by which comprehensive discrimination of a disease from among greater varieties of heart disease is carried out. The invention includes the following:
(1) An electrocardiogram (ECG) analysis device in accordance with the present invention analyzes an ECG. The ECG analysis device includes means for analyzing level of an ECG feature value, means for determining disease information relating to a patient's disease based on information including the feature value, and means for outputting both the feature value analysis result analyzed by the ECG feature value analyzing means and the disease information result determined by the disease information determining means.
   The user who obtains the results outputted by the outputting means can understand both feature value analysis result and the disease information result. Therefore, the user can promptly and easily carry out diagnosis of heart diseases based on the ECG.
(3) The outputting means in accordance with the present invention further displays a chart that relates the feature value analysis result to each portion of heart.
   The user who obtains the feature value analysis result can understand the feature value in connection with the related portion of the heart. Therefore, the user can promptly and easily carry out diagnosis of heart diseases diagnosis in connection with the corresponding portion of the heart.
(4) The outputting means in accordance with the present invention further displays the chart in a radar chart form that arranges each of the feature value analysis result at the corresponding portion of the heart.
   The user who obtains the feature value analysis result can visually and intuitively understand the feature value in connection with the related portion of the heart. Therefore, the user can promptly and easily carry out a comprehensive aiding of heart diseases diagnosis in connection with the corresponding portion of the heart.
(5) The outputting means in accordance with the present invention further outputs history of the feature value analysis result and/or history of the disease information result when outputting the feature value analysis result.
   The user who obtains the results outputted by the outputting means can visually and intuitively understand history of the disease information result and/or the history of the feature value analysis result (e.g. when the feature value is in an abnormal range) in connection with the related portion of the heart. Therefore, the user can easily understand the tendency of the patient's abnormal condition together with the feature value analysis result.
(6) The outputting means in accordance with the present invention further outputs history summary of the feature value analysis result.
   For example, the user who obtains the feature value analysis result can promptly understand the history summary of feature value analysis result after completing the ECG analysis. Therefore, the user can promptly and easily carry out diagnosis of heart diseases by the prompt understanding of the history.
(7) The feature value in accordance with the present invention is based on the constituent elements of an ECG including P wave, Q wave, R wave, S wave, ST segment, or T wave.
   The user who obtains the results outputted by the outputting means can understand one or more feature values that are based on the corresponding constituent elements of the ECG, including the P wave, the Q wave, the R wave, the S wave, the ST segment, or the T wave.
(8) The disease information determining means in accordance with the present invention determines the disease information based on the Minnesota code as an ECG classification reference.
   The user who obtains the results outputted by the outputting means can understand both the feature value analysis result and the disease information result based on the Minnesota code. Therefore, the user can promptly and easily carry out a comprehensive and collective aiding of heart diseases diagnosis based on both the ECG feature value level and the Minnesota code.
(9) The ECG analysis device in accordance with the present invention further outputting heartbeat-related information by sound and/or varying display style during analyzing the ECG.
   The user who utilizes the ECG analysis device can check the ongoing patient's heart beat condition regardless of the results outputted by the outputting means. The user can confirm that the ECG analysis device is running normally by the sound or varying display style even if the feature value is in a normal range and the display for the feature value does not vary much.
(10) The ECG analysis device in accordance with the present invention further outputting a warning signal when the analysis can not be executed during analyzing the ECG.
   The user who utilizes the ECG analysis device can promptly understand a situation of impracticable analysis (e.g. when an electrode is detached or device breakdown) by the warning signal.
(13) A method for analyzing an ECG in accordance with the present invention comprising the steps of analyzing magnitude of an ECG feature value, determining disease information relating to a patient's disease based on information including the feature value, narrowing down the candidates of disease information result based on the feature value analysis result, and outputting the narrowed disease information result candidates.
   The method is capable of outputting the candidates of disease information result which are limited by considering both the analyzed magnitude of feature value and the disease information result.
(14) A method for analyzing an ECG in accordance with the present invention comprising the steps of analyzing magnitude of an ECG feature value, determining disease information relating to a patient's disease based on information including the feature value, determining different disease information than the determined disease information by considering both the feature value analysis result and the determined disease information result, and outputting the determined different disease information result.
   The method is capable of outputting the different disease information result, which is not specified by the pre-determined disease information, by considering both the analyzed magnitude of feature value and the disease information result.
(15) A method for analyzing an ECG in accordance with the present invention comprising the step of analyzing the ECG by combining an algorithm for analyzing level of an ECG feature value and an algorithm for determining whether a patient's cardiac function is abnormal or not, which is based on information including the feature value.

The method is capable of discriminating from among specific types of heart diseases with comprehensiveness and less errors based on both the analyzed ECG feature value and the abnormality of patient's cardiac function.
The followings are definitions of the terms.

The term "feature value" in the present invention includes information such as an ECG waveform critical point value, a waveform start point value, a waveform end point value, a waveform segment point value, an amplitude value, a wave number value, a wavelength value, or information derived from those values (e.g. an interval value). In other words, the term "feature value" includes an identified value extracted from an ECG waveform, or information derived from the identified value.

The term "level of an ECG feature value" in the present invention includes information such as abnormality or severity of disease obtained from information relating to the ECG feature value. More specifically, the term "level of an ECG feature value" includes magnitude of the feature value, magnitude of the difference between an obtained feature value and common average (or standard) of feature value, or magnitude of the difference between an obtained feature value and a feature value determined as normal. Forms of presenting the "level of an ECG feature value" includes presenting numeric value relating to a feature value or presenting certain step-by-step scale defined based on magnitude of the feature value.

The term "information including the feature value" in the present invention includes information of the above-mentioned "feature value" or combination of the "feature value" and other information. For example, the other information includes information indicating noise contamination, electrode detaching, device breakdown, etc., or information inputted by user.

The term "disease information" in the present invention includes information representing a living body's disease condition such as heart diseases, an ECG classification reference including the Minnesota code, disease severity, or an observation (e.g. "ST elevation", arrhythmia, or normal range).

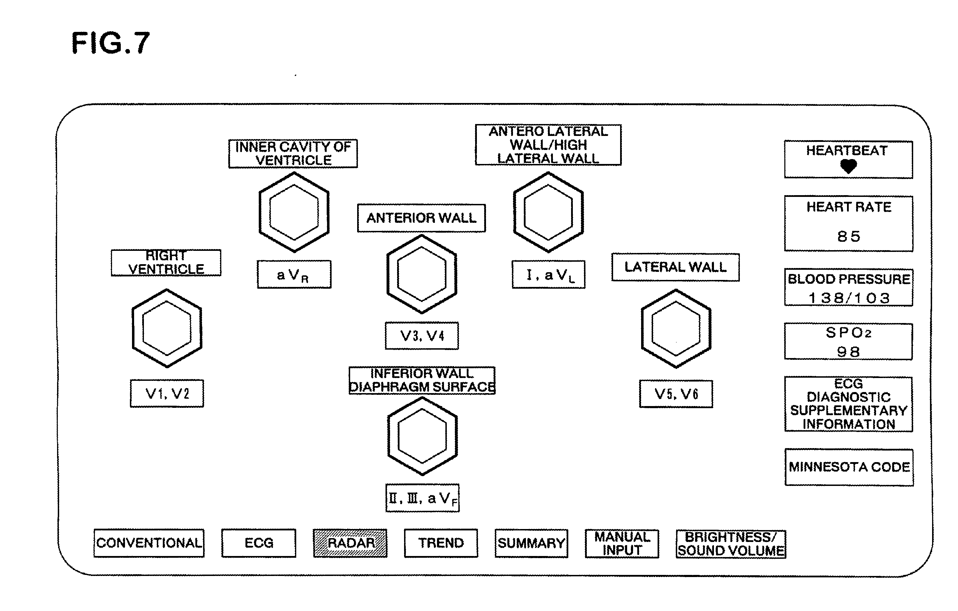

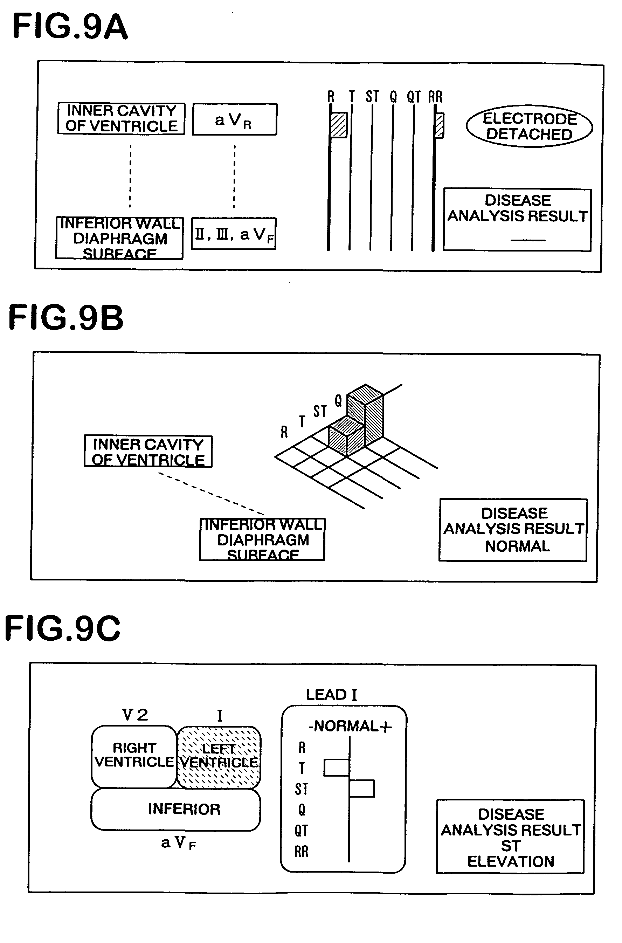

The term "chart display" in the present invention includes displayed points or displayed values in certain forms such as a listing, a diagram, a graph, a meter, or gage form. More specifically, the term "chart display" includes a bar graph (refer to Fig. 9A), a two dimensional graph, a three dimensional graph (refer to Fig. 9B), or a combination of diagram and a displayed value listing (refer to Fig. 9C), as well as the radar chart display (refer to Fig. 7, etc.).

The term "radar chart" in the present invention corresponds to a graph on which displayed points are displayed in a spider web format, and includes a polygonal-shaped display, such as a hexagon or pentagon, on which each displayed point is placed at a vertex of the polygonal shape.

The features of the present invention can be described broadly as set forth above. The structures and characteristics of the present invention will be apparent from the following detailed description of the invention together with those features, effects, and drawings.

### BRIEF DESCRIPTION OF THE DRAWINGS

Fig. 1 illustrates a processing overview of an ECG radar chart device according to an embodiment of the present invention.
Fig. 2A and 2B illustrate an annotated overview of an ECG radar chart screen (i.e., displaying an identified value level).
Fig. 3 illustrates an annotated overview of an ECG radar chart screen (i.e., displaying the Minnesota code).
Fig. 4 illustrates a program flowchart for process that generates an ECG radar chart executed by the ECG radar chart device.
Fig. 5 illustrates a program flowchart for an abnormality determining process for an identified value of RR interval.
Fig. 6 illustrates a program flowchart for process that determines the Minnesota code for code 6-4-1.
Fig. 7 illustrates an example of an ECG radar chart screen displaying values determined normal.
Fig. 8 illustrates an example of an ECG radar chart screen displaying a value determined abnormal.
Fig. 9A, 9B, and 9C illustrate other examples of an ECG radar chart.
Fig. 10 illustrates a hardware configuration example for the ECG radar chart device.
Fig. 11 illustrates an example of an ECG utilized by the CPU of the ECG radar chart device to obtain identified values.
Fig. 12 illustrates a screen example of ECG data in a trend mode.
Fig. 13A illustrates a diagram of stored ECG data.
Fig. 13B illustrates a diagram of stored identified value data.
Fig. 14 illustrates an overview of ECG radar chart system as another embodiment.
Fig. 15 illustrates a hardware configuration example for a device that sends an ECG radar chart.
Fig. 16 illustrates a hardware configuration example for a device that receives an ECG radar chart.
Fig. 17 illustrates a program flowchart for process of data transmission and reception executed by the ECG radar chart system.

### DESCRIPTION OF THE PREFERRED EMBODIMENTS

An ECG chart device as an embodiment of the present invention will be described below. The "ECG chart device" corresponds to the term "ECG analysis device". The following illustrates an ECG radar chart display processing based on the patient ECG data. According to the following embodiments, the user of the device can intuitively and easily carry out ECG interpretation in connection with specific heart diseases.

An overview of the embodiments, hardware configurations of devices, embodiments and structures corresponding to the terms in claims, and details of embodiments will be described below.

Table of Contents for the Embodiments
1. An overview of the embodiments
2. Hardware configurations of devices
3. Embodiments
4. Overview of ECG radar chart generating process
5. ECG radar chart generating process
6. Advantages of the embodiments
7. Other functions of the ECG radar chart device
8. Other embodiments

### 1. An overview of the embodiments

The ECG radar chart device 100 measures an ECG on the patient and displays the ECG in a radar chart form. The device is suitable for use in emergency medical arenas, in-ambulances, or in-hospitals. The ECG is obtained by measuring electric potential difference on the heart between two points on the patient's body. Therefore, the terms "ECG measurement" etc. used herein include the operations of measuring the heart's electrical potential etc.

### 2. Hardware configurations of devices

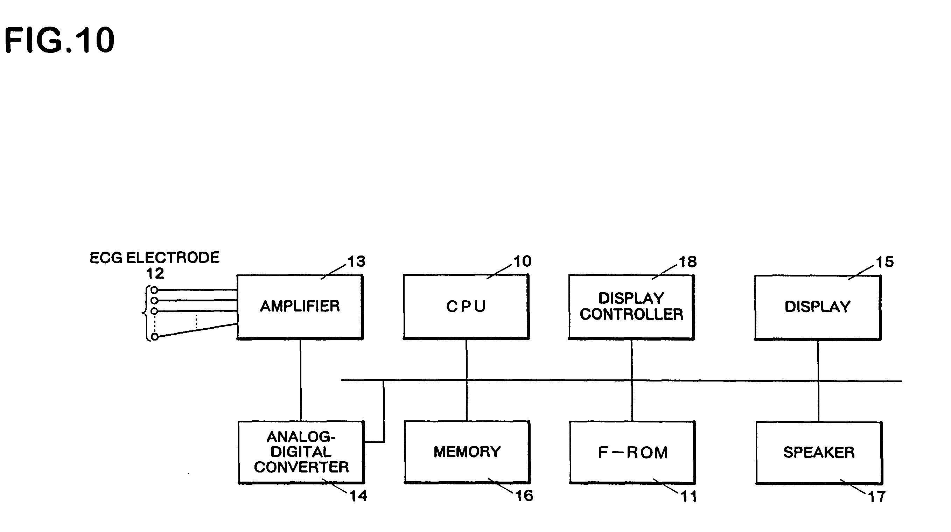

Fig. 10 illustrates a hardware configuration example of the ECG radar chart device 100 by use of a central processing unit (CPU). The ECG radar chart device 100 includes ECG electrodes 12, amplifier 13, analog-digital converter 14, CPU 10, Flash-ROM 11 (which corresponds to a rewritable read-only memory device from which recorded data can be electrically erased (e.g. the flash-memory), and will be described as "F-ROM 11"), memory 16, display controller 18, display 15, and speaker 17.

The ECG electrodes 12 are used for measuring a patient's heart's electric current. The amplifier 13 amplifies the heart's electrical current obtained through ECG electrodes 12. The CPU 10 controls operations of the ECG radar chart device 100, executes a process that converts data obtained from the heart's electric current to ECG data for displaying an ECG, and executes a process that displays an ECG radar chart. The F-ROM 11 stores a computer program for controlling the ECG radar chart device 100. The memory 16 acts as a storage area for data processing performed by the CPU 10. The display controller 18 controls the screen of display 15 in accordance with user operation.

In the embodiments, examples of operating systems for the ECG radar chart device 100 are Microsoft's Windows™ XP, NT, 2000, 98SE, ME, or CE. In alternative embodiments, the functions of the ECG radar chart device 100 are accomplished with hardware logic (not shown) without the use of a CPU. The hardware configuration or CPU configuration can be modified by well-known techniques by those skilled in the art.

### 3. Embodiments

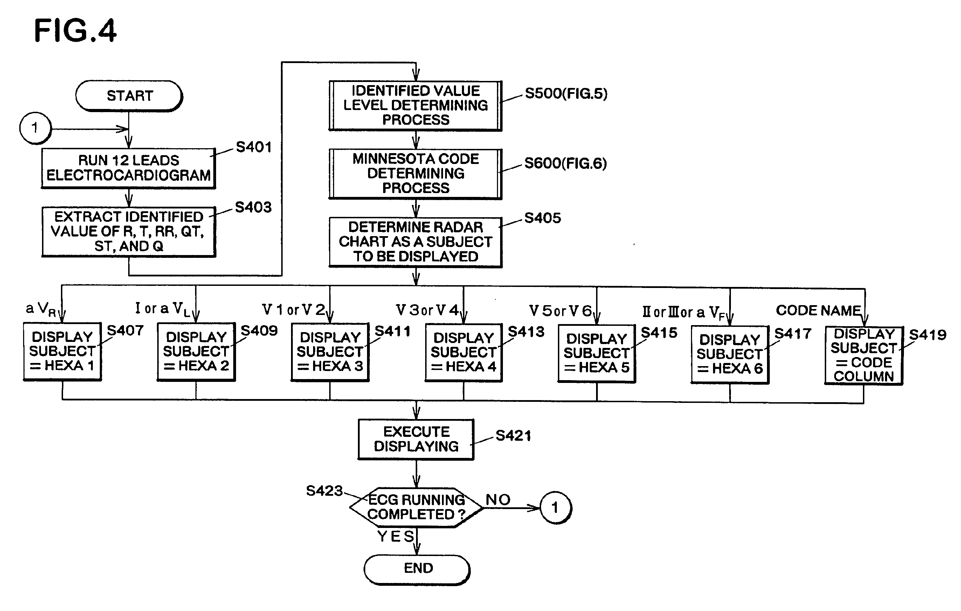

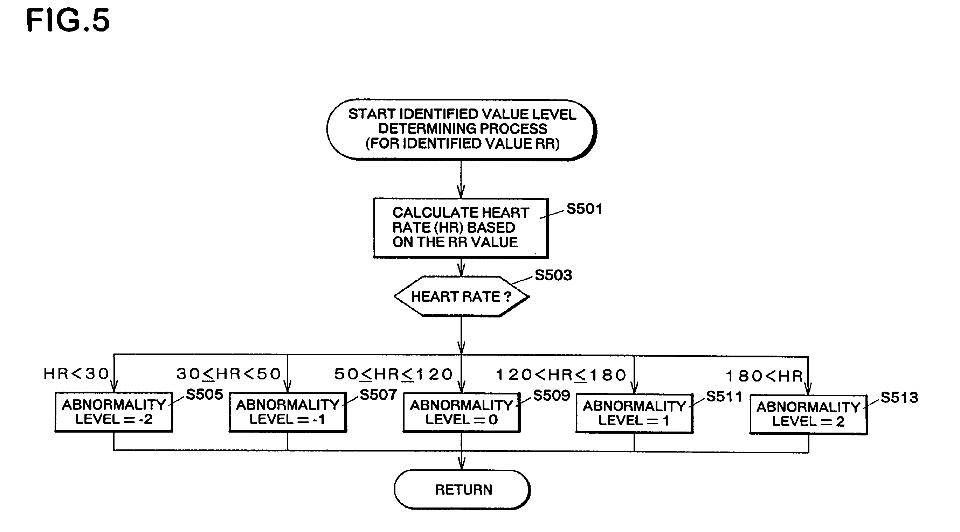

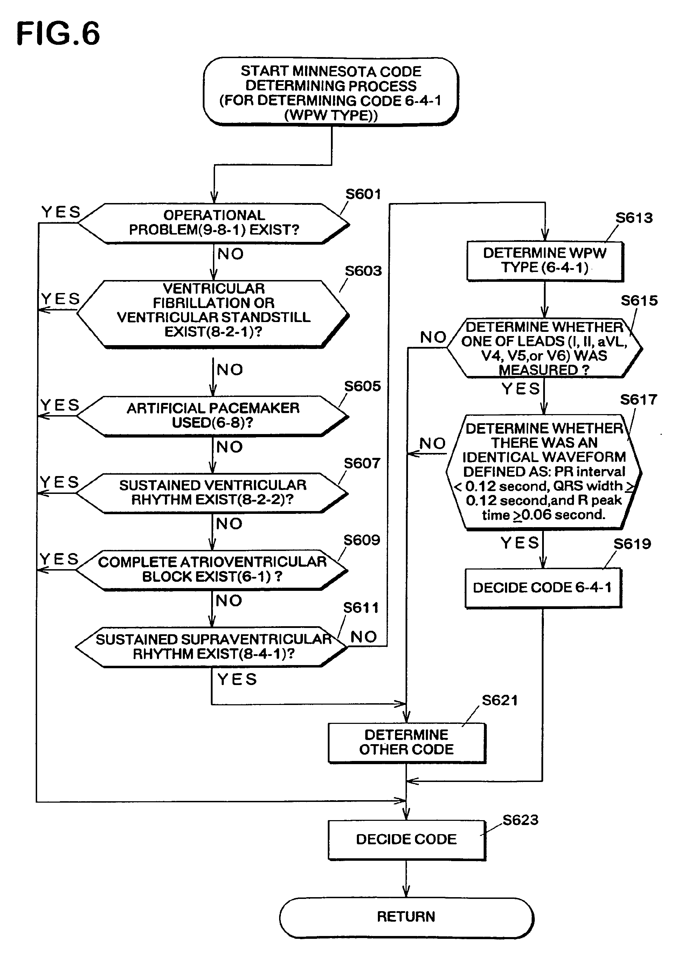

The "ECG analysis device" corresponds to the ECG radar chart device 100 illustrated in Fig. 10. The "a feature value analyzing means" or "an algorithm for analyzing level of a feature value" corresponds to the CPU 10 of ECG radar char device 100 executing the process that determines an identified value level at step 500 in Fig. 4 (refer to Fig. 5). The "disease information determining means" or "an algorithm for determining whether a patient's cardiac function is abnormal or not" corresponds to the CPU 10 executing the process that determines the Minnesota code at step 600 in Fig. 4 (refer to Fig. 6).

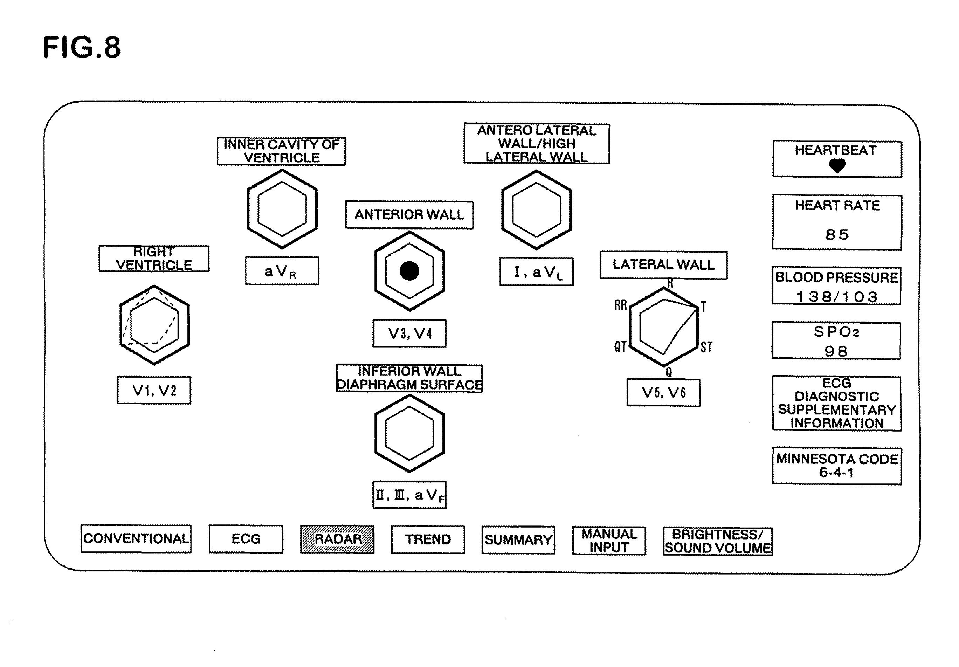

The "feature value" corresponds to the identified value extracted from the ECG waveform by the CPU 10 at step 403 in Fig. 4. The "feature value analysis result" corresponds to a radar chart representing identified values level (refer to Fig. 7 and Fig. 8). The "disease information result" corresponds to a column indicating name of the Minnesota code and/or a round mark (i.e., lead information or portion information of the heart) indicating lead(s) whose information is utilized for the Minnesota code determination (refer to Fig. 7 and Fig. 8).

The "outputting means" corresponds to the CPU 10 executing step 421 in Fig. 4.

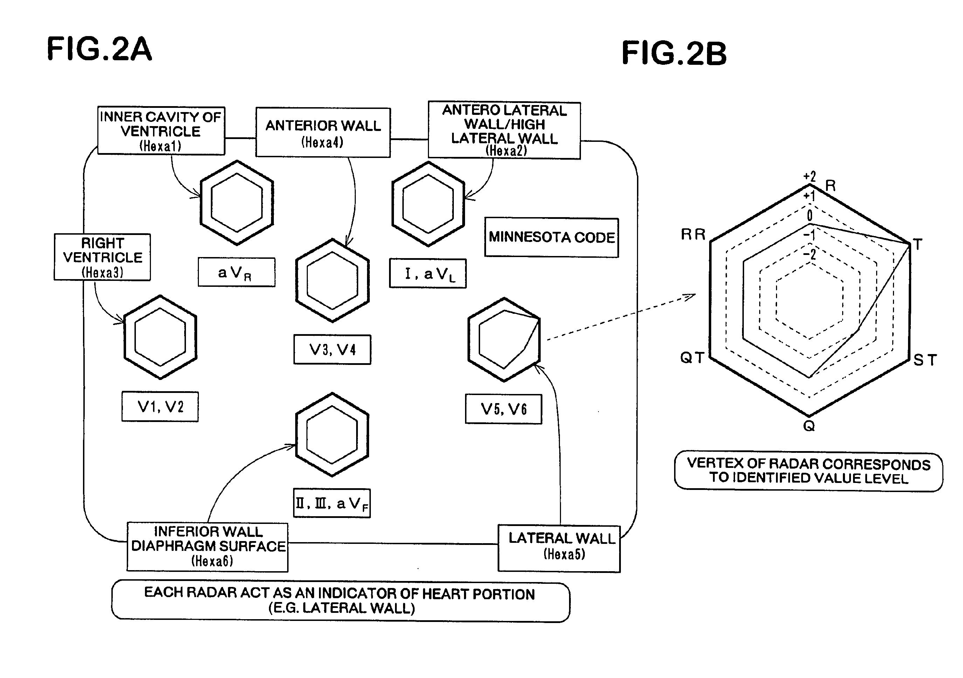

The "portion of the heart" corresponds to, as illustrated in Fig. 2 etc., the "antero lateral wall", the "high lateral wall" and/or the "anterior wall" as left portion of the heart, the "right ventricle" as right portion of the heart, the "inferior wall diaphragm surface" as lower portion of the heart, the "lateral wall" as front portion of the heart, or the "inner cavity of ventricle" as inner portion of the heart.

### 4. Overview of ECG radar chart generating process

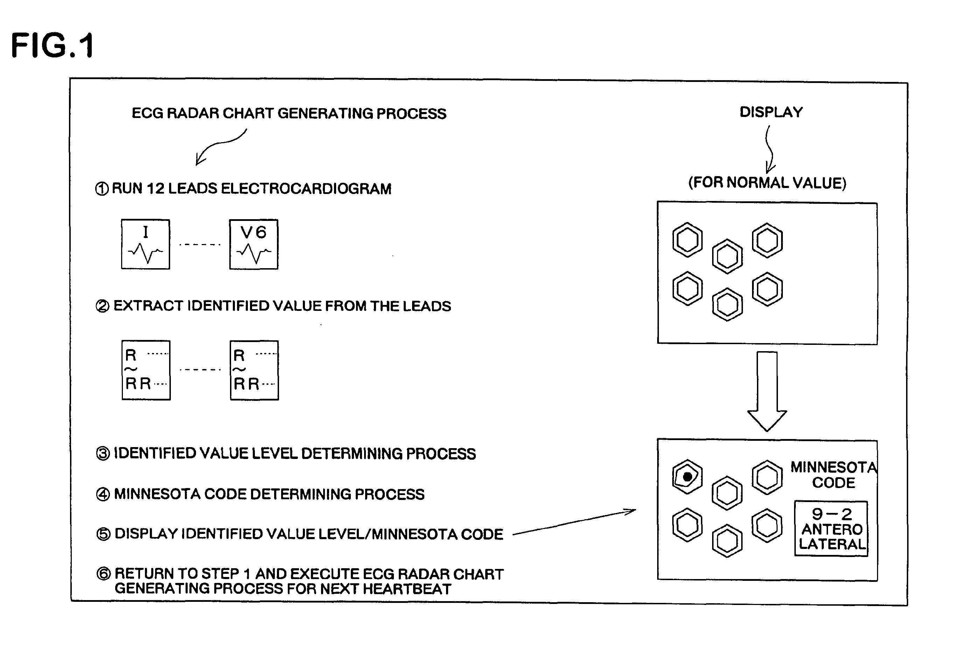

In this embodiment, as an example of the present invention, the CPU 10 of the ECG radar chart device 100 displays an ECG radar chart based on an ECG obtained from a patient. As an example, ECG radar chart generating process is executed once every heart beat. The sampling frequency for the ECG data is 125, 250, 500, 1000 Hz, or the like. In alternative embodiments, the timing for the ECG radar chart generating process can be modified by a well-known technique by those skilled in the art, such as at an every specific heart beat(s) other than the above-mentioned once every heart beat, or at other specified time periods. An overview of the ECG radar chart generating process will be described below together with Fig. 1, etc.

### 4-1. An overview of ECG radar chart generating process

Fig. 1 illustrates an overview of the ECG radar chart generating process executed by the CPU 10 of ECG radar chart device 100. The CPU 10 runs 12-lead ECG on a patient (step 1). At this step, a default screen (i.e. screen for normal values) is displayed. An overview of the screen contents will follow. The CPU 10 extracts identified values (which are based on P wave, Q wave, R wave, S wave, ST segment, or T wave) from the ECG waveform (step 2). The CPU 10 executes the process that determines the identified value level for every identified value (step 3). The CPU 10 determines the Minnesota code by utilizing information including the identified value (step 4). The identified value level determining process and the Minnesota code determining process will be described below. The CPU 10 displays the identified value level and the Minnesota code (step 5). The above-mentioned process is executed once every heart beat. At the next heart beat, the CPU 10 returns to step 1 and repeats the ECG radar chart generating process (step 6). The above-mentioned is an overview of the ECG radar chart generating process.

In alternative embodiments, the timing for the ECG radar chart generating process can be executed at specified time periods (e.g. every one second) other than the above-mentioned once every heart beat.

### 4-2. An overview of the ECG radar chart screen (displaying identified value levels)

Fig. 2 illustrates an overview of the ECG radar chart screen (displaying identified value levels). Fig. 2A illustrates hexagonal radar chart on the display with annotations. As illustrated in Fig. 2A, each hexagonal radar chart acts as an indicator for a portion of the heart corresponding to one or more of the 12 leads. More specifically, the (I, aVL) radar chart acts as an indicator for antero lateral wall and high lateral wall. The (V3, V4) radar chart acts as an indicator for anterior wall. The (V5, V6) radar chart acts as an indicator for lateral wall. The (II, III, aVF) radar chart acts as an indicator for whole of lower heart portion (e.g. inferior wall diaphragm surface). The (V1, V2) radar chart acts as an indicator for right ventricle. The (aVR) radar chart acts as an indicator for inner cavity of ventricle. Furthermore, each radar chart is placed at the corresponding physical portion of the heart on the schematic layout as illustrated in Fig. 2 (which corresponds to the term "arranges each of the feature value analysis result at the corresponding portion of the heart" in the claims). The classification as "12-lead ECG" according to each portion of the heart as mentioned above can be modified by well-known techniques by those skilled in the art. Also, the layout for the radar charts according to the corresponding heart portion can be modified by well-known techniques by those skilled in the art.

Fig. 2B illustrates an enlarged view of one radar chart with annotations. As illustrated in Fig. 2B, each vertex of a hexagonal radar chart acts an indicator for the identified value, i.e. step-by-step scale identified value level. More specifically, each point on the radar chart is adjusted on the basis of the value determined from a critical point, a start point, an end point, or etc., of the constituent element of the ECG waveform such as the P wave, the Q wave, the R wave, the S wave, the ST segment, or the T wave. In the embodiment, the values are six identified values such as the R value (i.e. R potential or R wave height), the T value (i.e., T potential or T wave height), the Q value (i.e. Q potential or Q wave height), the ST value (i.e. ST level), the QT value (i.e. QT interval), and the RR value (i.e. RR interval). When the identified value is in a normal range as illustrated in Fig. 2, the identified value level of "0" is displayed. On the other hand, according to the magnitude of abnormality, a point such as " - (minus)2", "-1", "+1", or "+2" is displayed. In the embodiments, as an example of the abnormality determination, when an identified value exceeds an upper limit of normal value, "+" is applied. On the other hand, when an identified value falls below a lower limit of normal value, "-" is applied. In addition, an absolute value of the identified value level represents magnitude of the abnormality. For example, in terms of the abnormality, an identified value level of "+2" means greater than that of "+1".

While some radar charts correspond to one lead, other radar charts correspond to several leads, as illustrated in Fig. 2. For a radar chart corresponding to several leads, if at least one value is identified as abnormal, a screen for the abnormal value can reflect the identified value level. In addition, if several values are identified as abnormal, a screen for the abnormal values can reflect the maximum or average of the identified values.

### 4-3. An overview of the screen (the Minnesota code displaying)

Fig. 3 illustrates an annotated overview of an ECG radar chart screen (i.e., displaying the Minnesota code). Compared with Fig. 2, a column for indicating the Minnesota code is additionally illustrated in Fig. 3.

As illustrated in Fig. 3, the Minnesota code as an ECG classification reference such as "7-7-1" (i.e., "Complete left bundle branch block") is displayed. The code name is an indicator for aiding in determination of patient's disease under an ECG measurement. In addition, a mark (e.g., a round mark) is displayed on the radar chart of "Hexa 5" as an indicator for heart portion(s) relating to the disease. When there is a lead that has a relation with a code determined by the Minnesota code determining process, the mark is displayed in order to indicate heart portion(s) associated with the lead. When there is no relation between a determined code and a lead, the mark displaying can be omitted.

Based on the ECG radar chart screen as mentioned above, the user of the ECG radar chart device 100 can intuitively and easily carry out ECG interpretation.

### 5. ECG radar chart generating process

The program flowchart for the ECG radar chart generating process will be described below together with Fig. 4, Fig. 5, Fig. 6, and the like. Fig. 4 illustrates the program flowchart to be executed once every heart beat. Therefore, the program execution in accordance with the flowchart illustrated in Fig. 4 will be repeated once every heart beat during the patient's ECG measurement.

Fig. 7 illustrates a default ECG radar chart screen displaying values determined normal. Each of the default ECG radar charts are formed in the shape of a regular hexagon. When a default ECG radar chart screen such as that illustrated in Fig. 7 is displayed, the screen indicates that each identified value of the 12 leads is within a normal range. The ECG radar chart device 100 provides several modes of screen variation that are selectable in accordance with user operation.

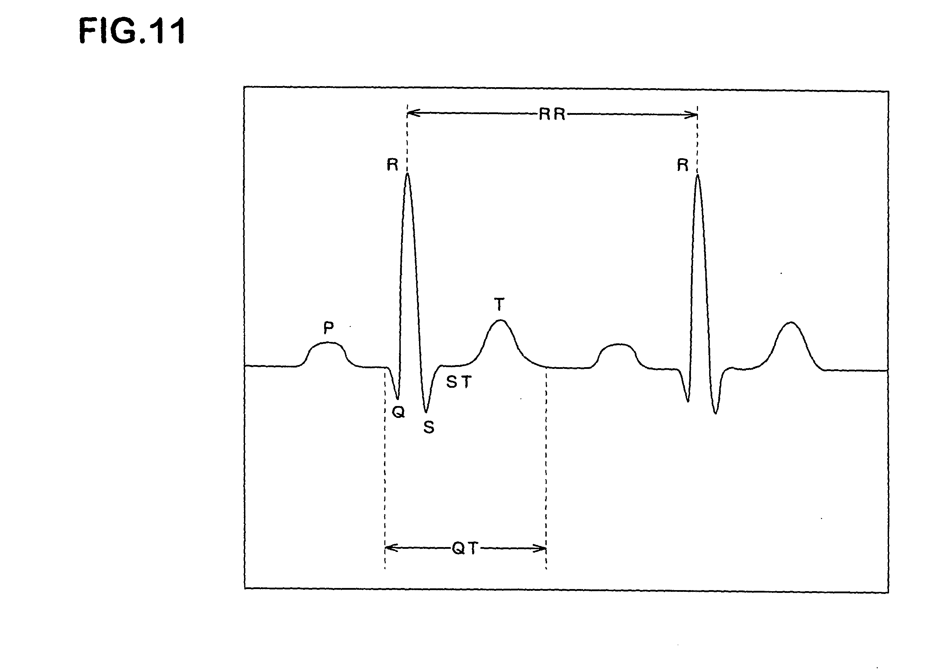

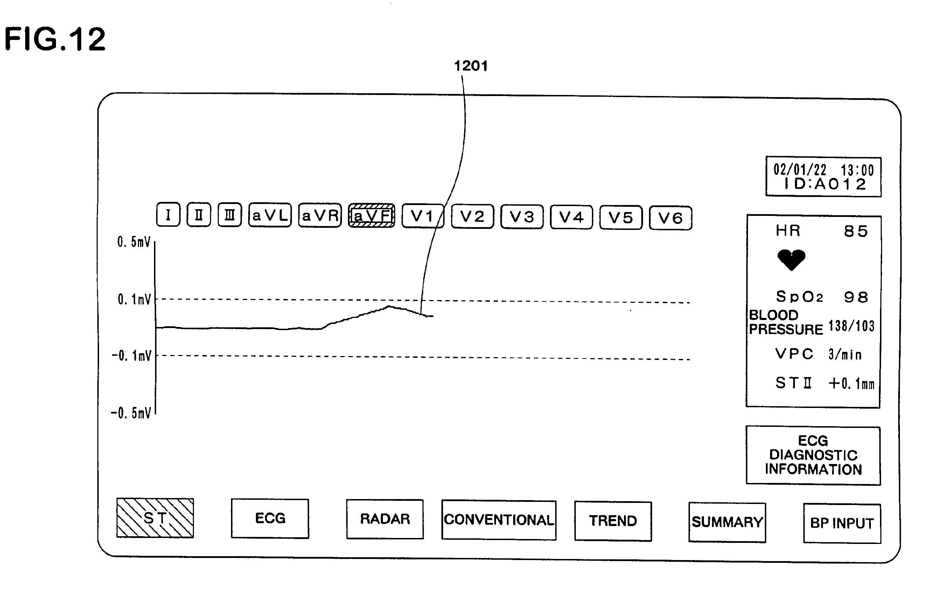

The first mode is an ECG radar chart mode as illustrated in Fig. 7 or the like. The second mode is an ECG mode for displaying an ECG (refer to Fig. 11 for an ECG display). In the ECG mode, the device displays all of the ECG data from the 12 leads all at once. In alternative embodiments, the device selects one or more leads, and displays the ECG data from the selected lead(s). The third mode is a trend mode. In the trend mode, the device displays the trends of each identified value based on each of the 12 leads in a graph form. Fig .12 illustrates a screen example for the trend mode. In Fig. 12, trend 1201 for lead aVF is displayed. For this trend, the identified values corresponding to each heartbeat are displayed. In an alternative embodiment, the average of identified values over regular time intervals is displayed. On the screen, the heart rate, blood pressure value, SP02 value, and supplementary information for ECG diagnosis, etc. are also displayed.

### 5-1. ECG radar chart generating process flowchart

The ECG radar chart generating process will be described below together with a flowchart. The CPU 10 of the ECG radar chart device 100 processes a 12-lead ECG by use of the ECG electrodes 12 placed on the patient's body and an amplifier 13, obtains ECG data based on the ECG, and stores the ECG data in memory 16 (or F-ROM 11) (step 401)(i.e., acting as data obtaining means). The CPU 10 extracts identified values (which are based on the P wave, the Q wave, the R wave, the S wave, the ST segment, or the T wave) from each of the 12 ECG leads, and stores the identified value data in memory 16 (or F-ROM 11) (step 403)(i.e., acting as identified value data computing means).

Fig. 11 illustrates an example of an ECG utilized by the CPU of the ECG radar chart device to obtain an identified value. For example, the CPU identifies one heart beat by identifying (or extracting) all or some of the following: P value (i.e. P potential or P wave height), Q value (i.e. Q potential or Q wave height), R value (i.e. R potential or R wave height), S value (i.e. S potential or S wave height), T value (i.e. T potential or T wave height), ST value (i.e. ST level), QT value (i.e. QT interval), or RR value (i.e. RR interval) as identified value data (or feature value data) from the ECG data. More specifically, at step 403, the CPU 10 extracts R value (i.e. R potential or R wave height), T value (i.e. T potential or T wave height), Q value (i.e. Q potential or Q wave height), ST value (i.e. ST level), QT value (i.e. QT interval), or RR value (i.e. RR interval) based on the P wave, the Q wave, the R wave, the S wave, the ST segment, or the T wave of ECG.

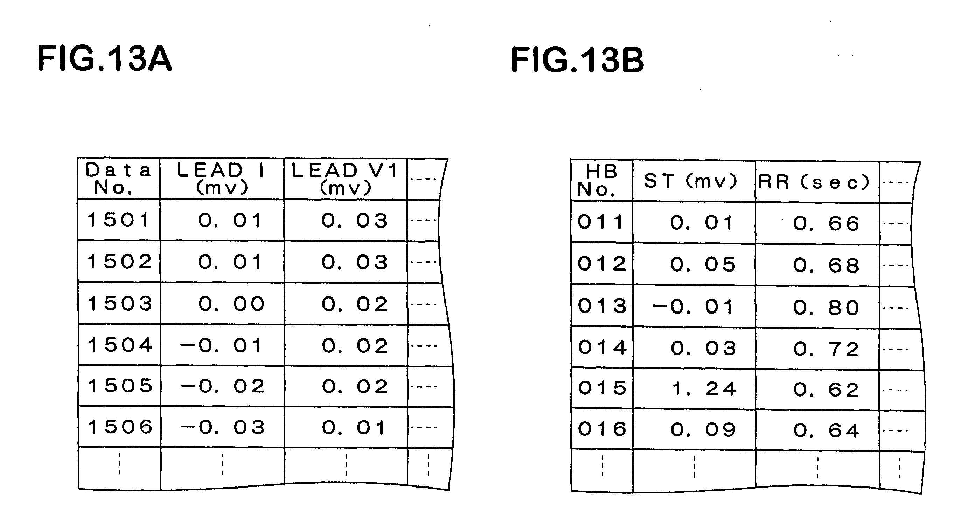

Fig. 13 illustrates a schematic diagram for stored contents of ECG data and identified value data. Fig. 13A illustrates examples of ECG data (which relate to lead I and lead V1), which are stored in memory 16 etc. at every ECG data sampling interval at step 401. Fig. 13B illustrates examples of identified value data (which relate to ST level and RR interval), which are stored in memory 16 etc. at every heart beat.

The CPU 10 executes the process that determines identified values level for the identified values extracted at step 403 (step 500). The CPU 10 then executes the process that determines the Minnesota code (step 600). The identified values level determining process and the Minnesota code determining process will be described together with flowcharts illustrated in Fig. 5 and 6.

The CPU 10 identifies lead(s) that is being associated with the obtained identified values level at step 500 and a name of the Minnesota code obtained at step 600 (and/or lead(s) that is being associated with the code name, or the like). The CPU 10 determines a radar chart as a subject to be displayed (step 405). For example, if the lead aVR relates to the subject to be displayed, the CPU 10 chooses radar chart Hexa 1 to display the subject (step 407) (positions for Hexa "X" is illustrated in Fig. 2). Similarly, the CPU 10 chooses the following radar charts: radar chart Hexa 2 when lead I or aVL relates to the subject to be displayed (step 409); radar chart Hexa 3 when lead V1 or V2 relates to the subject (step 411); radar chart Hexa 4 when lead V3 or V4 relates to the subject (step 413); radar chart Hexa 5 when lead V5 or V6 relates to the subject (step 415); radar chart Hexa 6 when lead II, III, or aVF relates to the subject (step 417); column for the Minnesota code when there is a determined Minnesota code name (step 419). The CPU 10 then executes the determined displaying process on the corresponding radar chart (step 421).

To determine the display subject at step 405, the CPU 10 can add position data indicating a display position (or data indicating each position of the radar chart points) to data indicating the determined identified value level at step 500 or the determined Minnesota code at step 600. In an alternative embodiment, the CPU 10 utilizes table data that correlates the data indicating each identified value with position data indicating the display position for the identified value.

After step 421, the CPU 10 determines whether the ECG measurement was completed (step 423). If the ECG measurement was not completed, the CPU 10 returns to step 401 and repeats the process. On the other hand, if the ECG measurement was completed, the CPU 10 completes the ECG radar chart generating process. At the determining process at step 423, the CPU 10 utilizes inputting information of an end of ECG measurement in accordance with a user's operation, or the like.

### 5-2. Identified value level determining process flowchart

The identified value level determining process executed by the CPU 10 at step 500 in Fig. 4 will be described below together with a flowchart illustrated in Fig. 5.

The identified value level determining process is to determine level of the identified values (which are based on the P wave, the Q wave, the R wave, the S wave, the ST segment, or the T wave) from each of the 12 ECG leads. In the embodiments, levels of the six identified values such as the R wave (i.e. R potential or R wave height), the T value (i.e. T potential or T wave height), the Q value (i.e. Q potential or Q wave height), the ST value (i.e. ST level), the QT value (i.e. QT interval), and the RR value (i.e. RR interval) are determined. The flowchart illustrated in Fig. 5 explains a process for determining level of the RR value as one of the six identified values, more specifically, abnormality level of heart rate (HR) from the RR value.

The following identified value level determining process will be described as a subroutine process executed at step 500 in Fig. 4.

As illustrated in Fig. 5, the CPU 10 calculates a heart rate (HR) based on the RR value obtained at step 403 in Fig. 4 (step 501 in Fig. 5). For example, the heart rate can be determined by the following equation, HR=60/RR interval (second). The CPU 10 determines an abnormality level based on the obtained heart rate (step 503). When the obtained HR was less than 30 (i.e., "HR < 30"), the CPU 10 determines an abnormality level of "-2" (i.e., bradycardia) (step 505). When the obtained HR was 30 or more and less than 50 (i.e., "30 ≦ HR < 50"), the CPU 10 determines an abnormality level of "-1" (i.e., bradycardia) (step 507). When the obtained HR was 50 or more and 120 or less (i.e., "50 ≦ HR ≦ 120"), the CPU 10 determines an abnormality level of "0" (i.e., normal value) (step 509). When the obtained HR was more than 120 and 180 or less (i.e., "120 < HR ≦ 180"), the CPU 10 determines an abnormality level of "1" (i.e., "tachycardia" or "rapid heart rate") (step 511). When the obtained HR was more than 180 (i.e., "180 < HR"), the CPU 10 determines an abnormality level of "2" (i.e., "tachycardia" or "rapid heart rate") (step 513).

The CPU 10 executes the above-mentioned process that determines identified value level for other five identified values (i.e., R, T, Q, ST, QT). The CPU 10 then executes the process that determines the Minnesota code at step 600 as illustrated in Fig. 4.

For other embodiments, abnormal values can be determined by using the magnitude of the difference between the identified values and normal values, or the difference between the identified value and an average identified value of the patient. Another identified value (e.g. PR interval etc.) instead of the identified value (which are based on a P wave, a Q wave, an R wave, a S wave, a ST segment, or a T wave) extracted at step 403 by the CPU 10 can be utilized for the determination of the identified value level.

In an alternative embodiment, the CPU 10 determines levels of the identified values by use of the identified values from past to present (e.g. The CPU 10 determines levels of the identified values by utilizing from the past two heart beats to processing heart beat, determines whether there has been more than 1 minute of ST elevation keeping more than 0.1 mV, or the like.) In that case, the CPU 10 accesses the identified values stored in memory 16.

When an ECG waveform contains abnormal noise due to patient movement during the ECG measurement process, it is usually difficult to extract the identified values accurately from the ECG at step 405 in Fig. 4. For example, the technology disclosed in Japanese Patent Laid-Open No. Hei 6-261871 can be utilized to obtain identified value data accurately by reducing such noise.

### 5-3. The Minnesota code determining process flowchart

The Minnesota code determining process flowchart executed by the CPU 10 at step 600 in Fig. 4 will be described below together with a flowchart illustrated in Fig. 6.

The Minnesota code determining process is to determine code(s) by utilizing information including the identified values (which are based on the P wave, the Q wave, the R wave, the S wave, the ST segment, or the T wave) from each of the 12 ECG leads. The Minnesota code is commonly used as an ECG classification reference. As an example, a modification of an ECG automatic analysis computer program using the Minnesota code can be applied in the embodiments.

The flowchart illustrated in Fig. 6 explains a process for determining "code 6-4-1 (Wolff-Parkinson-White (WPW) type)" as a sub-classification of the Minnesota code (which commonly includes 9 classifications).

As a rule of the Minnesota code, the other codes (i.e., in a sequential order of code 9-8-1, 8-2-1, 6-8, 8-2-2, 6-1, and 8-4-1) have been preferentially determined before determining the WPW type. In addition, when the other code(s) is determined to be applied, the other code(s) should be final outcome without subsequently determining the WPW type. When determining the code 8-4-1 can not be applied, the CPU 10 then determines the code 6-4-1 (WPW type). However, when determining the code 8-4-1 can be applied, the CPU 10 then determines code(s) other than the code 6-4-1.

The following Minnesota code determining process will be described as a subroutine process executed at step 600 in Fig. 4.

The CPU 10 determines whether a problem with the operation existed (step 601 in Fig. 6). If a problem with the operation existed, the CPU 10 decides "9-8-1" as a code (step 623). The problem with operation includes a situation where the code determination can not be carried out because of technical reason such as noise contamination, electrode detaching, or device breakdown. For example, the presence of noise contamination can be determined by the CPU executing a software program with digital filter etc. In alternative embodiments, when the device is out of order, a user can input the code into the device after visually evaluating the device breakdown.

If no problem with the operation existed, the CPU 10 determines whether a ventricular fibrillation or ventricular standstill existed (step 603). If a ventricular fibrillation or ventricular standstill existed, the CPU 10 decides "8-2-1" as a code (step 623). For example, the presence of ventricular fibrillation can be determined by the CPU executing a software program with frequency analysis function, etc. The presence of ventricular standstill can be determined by the CPU executing a software program that measures whether more than 5 seconds of the QRS wave has not been detected.

If no ventricular fibrillation or ventricular standstill existed, the CPU 10 determines whether an artificial pacemaker was used (step 605). If the artificial pacemaker was used, the CPU 10 decides "6-8" as a code (step 623).

If the artificial pacemaker was not used, the CPU 10 determines whether a sustained ventricular rhythm existed (step 607). If the sustained ventricular rhythm existed, the CPU 10 decides "8-2-2" as a code (step 623). For example, the CPU can decide the presence of sustained ventricular rhythm by determining whether waves that are defined as "heart rate (per minute) is 50 or less, QRS width is 0.12 second or more, and there is no P wave that accompanies with QRS wave" have existed continuously.

If no sustained ventricular rhythm existed, the CPU 10 determines whether a complete atrioventricular block existed (step 609). If the complete atrioventricular block existed, the CPU 10 decides "6-1" as a code (step 623). For example, the CPU can decide the presence of complete atrioventricular block when determining a case in which a heart atrium rate is more than heart ventricle rate (i.e., "heart atrium rate" > "heart ventricle rate") and the heart ventricle rate is less than 60 (i.e., "heart ventricle rate" < 60) at any of the leads.

If no the complete atrioventricular block existed, the CPU 10 determines whether a sustained supraventricular rhythm existed (i.e., code "8-4-1") (step 611). If the sustained supraventricular rhythm existed, the CPU 10 executes determination for other code(s) (step 621). For example, the CPU can decide the presence of sustained supraventricular rhythm when determining a case in which QRS width is less than 0.12 seconds, P wave loss or presence of abnormal P wave, and rhythm is regular.

For the determination for other code(s) at step 621, the CPU 10 can execute a computer program (not shown) that is designed based on the Minnesota code rule.

If no sustained supraventricular rhythm existed, the CPU 10 executes determination for the WPW type (i.e., code 6-4-1) (step 613). The CPU 10 determines whether at least one of the leads (i.e., I, II, aVL, V4, V5, or V6) was measured (step 615). If the leads were not measured, the CPU 10 determines other code(s) (step 621).

If one of the leads (i.e., I, II, aVL, V4, V5, or V6) was measured, the CPU 10 determines whether there was an identical waveform that is defined as PR interval is less than 0.12 second (i.e. PR interval < 0.12 second), QRS width is more than 0.12 second (i.e. QRS width > 0.12 second), and R peak time is more than 0.06 second (i.e. R peak time > 0.06 second) (step 617). If there was no identical waveform defined as the above-mentioned, the CPU 10 determines other code(s) (step 621).

The term "identical wave" means a waveform derived from single lead. For example, when lead I and lead V4 were measured, determination with an identical waveform should be done based on the values derived from only lead I such as PR intervals, QRS width, and R peak time. Therefore, determining based on both PR interval of lead I and QRS width of lead V4 does not mean "determination with an identical waveform".

If there was an identical waveform that is defined as PR interval is less than 0.12 second, QRS width is 0.12 second or more, and R peak time is 0.06 or more, the CPU 10 decides 6-4-1 (WPW type) as a code (step 619). The CPU 10 then obtains the code (step 623).

The CPU 10 executes the above-mentioned process that determines Minnesota code for other code(s). The CPU 10 then executes the remaining processes from step 405 in Fig. 4 for determining radar chart(s) as display target(s).

The processes of step 601, 603, 605, 607, 609, 611, 615, and/or 617 are described as examples. In alternative embodiments, the Minnesota code can be determined by utilizing information including an identified value (which is based on the P wave, the Q wave, the R wave, the S wave, the ST segment, or the T wave) from each of the 12 ECG leads, hardware (not shown) for detecting living body's information, or the like.

In addition, the Minnesota code determining process for the other code(s) can be done by utilizing information such as an identified value (which is based on the P wave, the Q wave, the R wave, the S wave, the ST segment, or the T wave) from each of the 12 ECG leads. The following is an example of process flow for determining the Minnesota code classification.
- code 8: arrhythmia (or an irregular heartbeat)
- code 6: atrioventricular conduction disturbance
- code 7: intra-ventricular conduction disturbance
- code 1: Q type and QS type
- code 4: ST junction and ST deviation
- code 5: abnormal T wave
- code 9-2: ST elevation
- code 3: high amplitude R wave
- code 2: QRS axis deviation
- code 9: miscellaneous items
- code 0: normal range

More than two codes can be applied to a certain disease.

### 5-4. Identified value level and the Minnesota code display

Fig. 8 illustrates an example of an ECG radar chart screen displayed by a displaying process at step 421 in Fig. 4 when an identified value was determined abnormal (refer to step 500 in Fig. 4 and Fig. 5) and the Minnesota code was determined (refer to step 600 in Fig. 4 and Fig. 6).

Fig. 8 illustrates a case in which T value (i.e. T potential or T wave height) and ST value (i.e. ST level) of lead V5 or V6 are determined as abnormal levels by displaying a radar point of Hexa 5 radar chart with a modified level that relates to the abnormal value (i.e. displaying with modified outline of a radar chart). More specifically, the CPU 10 stores ECG radar chart data (i.e. screen display data) in memory 16 (or F-ROM 11). The ECG radar chart data is used to display a radar point with a modified level, which relates to the abnormal value, on the display subject radar chart (Hexa "X"). The CPU 10 then displays the level-modified ECG radar chart. In addition, the CPU 10 displays the decided code of "Minnesota code 6-4-1" and lead name (which corresponds to "heart portion") of "Hexa 4" that was used as a basis for deciding the code by a warning display (i.e. a round mark). As an example of processing at step 617 in Fig. 6, Fig. 8 illustrates the code "6-4-1" with an warning display at position of the Hexa 4 under a case in which an identical waveform of lead V4 was defined as PR interval is less than 0.12 second (i.e., PR interval < 0.12 second), QRS width is more than 0.12 second (i.e. QRS width > 0.12 second), and R peak time is more than 0.06 second (i.e. R peak time > 0.06 second).

As mentioned above, the ECG radar chart device 100 displays the ECG radar chart based on the patient's ECG data. Therefore, the user of the ECG radar chart device 100 can intuitively and easily carry out interpretation of the ECG. As mentioned above, one feature of the embodiment is that the device displays a radar chart as an indicator of the portion of the heart corresponding to each lead. Another feature of the embodiment is to display each vertex on the radar chart corresponds to an identified value as well as a decided Minnesota code and lead name (i.e. index of heart portion) that was used as a basis for deciding the code.

The above-mentioned selection of lead for generating a radar chart, displaying the heart portion, criteria for determining identified value level, criteria for determining the Minnesota code, an algorithm for the process that generates ECG radar chart, arrangement of the radar chart, or arrangement of the warning display are described for illustrative purposes, and therefore, can be modified by those skilled in the art. For example, in the embodiments, the Minnesota code determining process is executed after the process that determines the identified value level (refer to step 500 and 600 in Fig. 4). In alternative embodiment, the Minnesota code determining process can be executed before the process that determines the identified value level. Also, those two process can be executed in parallel or more than two CPU can execute those two process in parallel (i.e. parallel processing).

### 6. Advantages of the embodiments

In the embodiment, the CPU 10 of the ECG radar chart device 100 displays the identified ECG value obtained from the 12 leads by placing the value so that the value acts as an indicator for the portion of the heart corresponding to each lead. Furthermore, the ECG radar charts representing level of the identified values are placed at the corresponding portion of the heart on the schematic layout, more specifically, displayed by arranging the identified values so as to associate with physical relationship of the heart portions (refer to Fig. 7 and 8).

Therefore, the user of the ECG radar chart device 100 can visually and intuitively understand each identified value level of a patient's ECG in connection with the related portion of the heart. In addition, since the level of the identified values are displayed by use of a radar chart, the user can collectively and simultaneously understand the variation in the identified values, or balance (or proportion) among the identified values derived from each of the leads.

In the embodiments, the CPU 10 of ECG radar chart device 100 outputs results of both the identified value level determining process and the Minnesota code determining process simultaneously (refer to Fig. 7 and 8).

The user can understand both determined level of the identified values and determined Minnesota code. Therefore, the user can promptly and easily carry out diagnosis of heart diseases based on both identified value determined as an abnormal level and the determined Minnesota code.

As illustrated in Fig. 8, the CPU 10 of ECG radar chart device 100 displays the code name as well as, when identifiable, heart portion (which is associated with each of the leads) from which the determined code is derived (refer to the round mark in Fig. 8). (The analysis result outputting means outputs the disease information result determined by the disease information determining means by corresponding to a lead name (or a heart portion) that was used as a basis for deciding the disease information result.) Therefore, compared to a conventional ECG automatic analysis computer program that is for displaying the Minnesota code name only, the program according to the present invention enables the ECG radar chart 100 to represent determined Minnesota code in details to the user by indicating the code as well as the heart portion (or indicating a lead).

Generally, the identified value level determining process and the Minnesota code determining process can be executed by independent methodology respectively. Therefore, for example, when an identified value is determined as normal, the Minnesota code may be determined as abnormal. Adversely, when an identified value is determined as abnormal, the Minnesota code may be determined as normal. In those cases, the ECG radar chart device 100 utilized a combination of the process that determines identified value level and the process that determines the Minnesota code. Therefore, it is advantageous for users to carefully assess the presence or absence of heart diseases.

Those mentioned above are advantages of the embodiments. The advantages are based on the unique features of the present invention such as displaying a wide variety of information derived from several leads and several identified values as a radar chart of identified values as well as displaying the Minnesota code so that users can easily and intuitively understand the wide variety of information.

### 7. Other functions of the ECG radar chart device

In addition to the above-mentioned ECG radar chart generating process, examples of optional functions of the ECG radar chart device 100 will be described below.

### 7-1. Display of heart beat condition

The ECG radar chart device 100 displays a specific flashing symbol (or mark) in order to show a heart beat condition (which corresponds to the term "heart beat related information") (which corresponds to the term "varying display style"). More specifically, the CPU 10 processes a display of the flashing heart mark according to the heart rhythm measured, as illustrated in Fig. 7.

The user can confirm that the ECG radar chart device 100 is running normally, and can also check the patient's heart beat condition. In an alternative embodiment, the device outputs a specific sound (e.g. bleep sound) from the speaker 17 according to the heart rhythm, in conjunction with the flashing mark or instead of the flashing mark.

### 7-2. Display of measurement history during ECG measurement

During the ECG radar chart generating process, the ECG radar chart device 100 can optionally display historical data of the patient's identified value levels (which corresponds to the term "history of the feature value analysis results"). More specifically, the CPU 10 can display the history of an identified value level that is in an abnormal range by dotted lines, such as on the radar chart of "right ventricle" illustrated in Fig. 8.

The user can ascertain that a patient may have a particular type of heart disease by checking the history of the identified value level. In an alternative embodiment of the display of identified value level history, any chart tracks instead of dotted lines can be applied to display the history. For example, the device can display the tracks in a different color from that being used for the ongoing identified value level. In another alternative embodiment, the device can display an additional specific mark next to a radar chart, corresponding to a lead relating to a determined abnormal value. In another alternative embodiment, instead of displaying all of the abnormal value history, the device can display the history only when the number of times determined as abnormal value exceeds a certain threshold number (e.g. when the number of times a determined value becomes abnormal exceeds three times).

In alternative embodiments, with or without displaying the identified value level history, the device can display the Minnesota code history (which corresponds to "history of the disease information result").

### 7-3. Display of history summary after ECG measurement

The ECG radar chart device 100 displays a history summary of the identified value levels (which corresponds to the term "history summary of the feature value analysis results") after completing the ECG measurement. The CPU 10 displays the history by utilizing the identified value data stored in memory 16 (or F-ROM 11). More specifically, the CPU 10 can optionally display a time-varied ECG radar charts in fast-forward on display 15 after completing the ECG measurement in accordance with user operation. The history can be represented by playing the screen illustrated in Fig. 8 in a frame advance (simplified moving picture playback).

The doctor in a hospital to which a patient is transferred can promptly understand the general trends for the patient's identified value level history. The function of the fast-forward playing of the identified value can be executed by extracting and displaying abnormal values only. In order to display the "history summary of the feature value analysis results," the device can display all the radar charts that correspond to the abnormal values determined as freeze-frame pictures rather than by fast-forward playing.

### 7-4. Warning for impracticable analysis

The ECG radar chart device 100 displays a certain warning (which corresponds to the term "warning signal") during ECG radar chart generating process when an ECG electrode 12 is detached from the patient or when trouble occurs in display processing (which corresponds to the term "a condition in which the analysis can not be executed"), or the like. More specifically, the CPU 10 displays a warning message stating "electrode detached" etc., on display 15 (refer to Fig. 9A).

The user who sees the warning can promptly understand that the ECG radar chart generating process has been interrupted by the trouble. In alternative embodiments, in order to draw the user's attention to the display, the CPU 10 changes the color of the whole display or the color of part of the display, or outputs a warning sound (e.g. an alarm sound).

### 8. Other embodiments

### 8-1. Disease analysis

In the embodiments, the Minnesota code is illustrated as the "disease analysis result". In alternative embodiments, the device can display an observation name and/or disease name based on the Minnesota code.

In the embodiments, the Minnesota code determining process is illustrated as a process of the "disease information determining means" (Fig. 6). In alternative embodiments, other ECG classification references can be used as a process of the "disease information determining means". For example, as the other ECG classification references, the determining process can be executed based on information other than the identified values, or information including the identified values. In alternative embodiments, a combination of two or three or more ECG analysis methodologies in combination can be used as a process of the "disease information determining means". For example, the other ECG classification reference as a process of the "disease information determining means" includes the Lown classification, Sokolow-Lyon ECG criteria, Romhilt-Estes criteria, or the like.

As a process of the "disease information determining means", the CPU can execute a matching process that compares ongoing measured ECG waveform with a model pattern of abnormal ECG waveform. More specifically, certain ECG waveform that can be associated with specific heart disease is defined as an abnormal waveform pattern. The CPU can determine the similarity between information based on ongoing measured ECG and the abnormal waveform pattern. If high similarity was obtained, the CPU can display the associated heart disease name as a determination result.

### 8-2. Pathologic analysis based on a combination of the feature value analysis means and disease information determining means

In the embodiments, identified values level as the feature value analysis result and the Minnesota code as the pathologic analysis result are outputted. In alternative embodiments, a pathologic analysis based on a combination of the feature value analysis means and disease information determining means can be executed as described below.

The first technique for the pathologic analysis based on a combination of the feature value analysis means and disease information determining means is to narrow down candidates of the disease analysis result based on the feature value analysis result.

More specifically, the CPU 10 of ECG radar chart device 100 narrows down results of the process that determines the Minnesota code based on results of the process that determines the identified value level. For example, when the Minnesota code determining process decides code 9-8-1 (i.e., operational problems that interfere with the code determination), output candidates are thought to include several results such as "electrode detached" or "noise contamination" (refer to step 601 in Fig. 6). The CPU 10 then utilizes information of determined identified values level on radar charts. More specifically, when no identified value level is displayed on the radar chart, the CPU 10 can decide that there is possible electrode detaching. On the other hand, when an identified value is displayed as an abnormal level on the radar chart, the CPU 10 can decide that there is possible noise contamination. The CPU 10 then outputs the narrowed down result. The term "narrowing down the candidates of disease information result" includes selecting one final candidate from disease information candidates etc., or reducing the number of disease information candidates and selecting the reduced candidates.

The second technique for the pathologic analysis based on a combination of the feature value analysis means and disease information determining means is to determine different disease information than pre-determined disease information by considering both the feature value analysis result and the determined disease information result.

More specifically, the CPU 10 outputs different disease information (i.e. disease name, diagnostic name, or the like) than pre-determined Minnesota code based on result(s) of the process that determines identified value level and result(s) of the process that determines Minnesota code. The second technique will be described below by using "long QT syndrome" as an example of the different disease information. For example, the "long QT syndrome" is characterized by prominent long QT and frequent occurrence of ventricular arrhythmia. Therefore, when level of QT interval indicates certain abnormal value on a radar chart (i.e. long QT) and determined Minnesota code is a code relating to "ventricular arrhythmia" (i.e. code 8-1-2 etc.), the CPU 10 can output a result presenting that the patient may have "long QT syndrome". The "long QT" is characterized by that QTc (i.e. corrected QT time) is 450 (or 440, 460) mili seconds or more where QTc is determined by the following equation, QTc=QT interval/(route RR interval (i.e. √ (RR interval)).

As above-mentioned, the outputted "different disease information" based on result of the process that determines identified value level and result of the process that determines the Minnesota code includes the diagnostic name that can not be determined by the process that determines the Minnesota code. In alternative embodiments, the outputted "different disease information" includes diagnostic name derived from different Minnesota code than pre-determined Minnesota code.

The pathologic analysis based on the combination of the feature value analysis means and disease information determining means enables the device to output further identified diagnostic name from the disease analysis results based on the Minnesota code, or to output other diagnostic name that is not limited to the disease analysis results.

### 8-3. Embodiments of ECG radar chart display

In the embodiments, the CPU 10 of ECG radar chart device 100 displays the ECG radar chart on display 15 based on the ECG data (refer to Fig. 7, 8). As alternative embodiments for chart display based on the ECG data, display methods such as those illustrated in Fig. 9 are applied. An overview of each display method will be given.

Fig. 9A illustrates a screen in which ECG data are displayed in a bar graph form. As illustrated in Fig. 9A, varying appearances of the R potential and the RR interval as identified values of the lead "aVR," relating to the condition of inner cavity of ventricle are displayed.

Fig. 9B illustrates a screen in which the ECG data are displayed in a three dimension graph form. As illustrated in Fig. 9B, varying appearances of the ST level and the Q potential of the lead "aVR," relating to the conditions of inner cavity of ventricle are displayed. In addition, the display represents that a disease analysis result is determined as normal.

Fig. 9C illustrates a screen in which the ECG data are displayed on a heart diagram, with a listing of identified values provided. As illustrated in Fig. 9C, the identified values (i.e. T potential and ST level) of the lead "I" relating to conditions of left ventricle are displayed as the determined abnormal values. In addition, the display represents that a disease analysis result is "ST elevation".

In any of the display method as mentioned above, it is desirable that the device displays each identified value by associating them with the related portion of the heart, so that the user of the device can promptly understand necessary information that should be interpreted from the ECG waveforms.

In alternative embodiments, a heart sketch (or heart diagram) can be displayed as a background (i.e. background picture) on the display in order to associate the ECG analysis result with the related portion of the heart.

### 8-4. Embodiments of outputting identified value level and the Minnesota code

In the embodiment, a screen representing feature value analysis result and disease information result is illustrated as an example of the "result outputting". In alternative embodiments, the feature value analysis result and/or the disease information analysis are treated in other applications such as data to be outputted, information to be outputted in memory card, CD-ROM, etc., information to be outputted in connection means (e.g. telephone lines, wireless communication, the Internet, wire communication, infrared data communication, mobile phone, Bluetooth, PHS, or the like), audio output, or printed hard copy output. For example, an observation name from the Minnesota code can be read by a computer as an audio warning. In the embodiments, the disease information result is displayed on display 15 as one device. In alternative embodiments, the "outputting means" can output the feature value analysis result and the disease information result separately. More specifically, the feature value analysis result and the disease information result are displayed on separate displays (not shown).

In the embodiments, when an identified value is in an abnormal range, a radar chart shape is changed by moving the point indicating each identified value toward a position corresponding to certain level in order to draw the user's attention to the value determined as abnormal (refer to Fig. 8). The following techniques are also applicable to draw the user's attention to the value.

The other technique for drawing the user's attention to an abnormal value is to change the color of the radar chart (or a part of the radar chart) or the color of the radar chart point when a value is determined as abnormal. The color change can be executed, for example, by the following method: The device displays a radar chart in black color etc. when an identified value is normal. If the identified value becomes abnormal, the device changes the color of radar chart to red color or the like. In alternative embodiment, the device changes the color of radar chart according to the magnitude of abnormality (i.e. green, yellow, red, etc. (not shown)).

In the embodiments, the device shows a decided Minnesota code to users by displaying the code as well as displaying a round mark near a lead that is used as a basis for deciding the code on display 15. The following technique is also applicable to alert users to the result of determined Minnesota code.

The technique for alerting users to the result of determined Minnesota code is to change the color of screen (all or a part of the screen) or the color of the radar chart (all or a part of the radar chart) based on the result of determined Minnesota code. More specifically, the change of radar chart can be executed based on the following reference (not shown) : yellow = ventricular premature contraction (VPC) as the determined Minnesota code, purple = WPW syndrome, red = tachycardia or rapid heart rate, light blue = right bundle branch block, blue = left bundle branch block, ash color = noise, or the like (the color can be modified).

The following methods are also applicable to alerting an abnormal value and/or alerting the Minnesota code.

The first alert method is to display a screen (all or a part of the screen) or a radar chart (all or a part of the radar chart) with blinking (i.e. flashing), or with pop-up action (e.g. zoom displaying). For example, the device displays a radar chart with blinking that corresponds to a lead including an abnormal value. In alternative embodiments, when a disease of cardiac hypertrophy is determined, the device displays a radar chart with an enlarged view (or zoom view) that corresponds to a lead associating with the disease.

The second alert method is to change the color of screen or radar chart according to the disease severity (i.e. abnormality of identified values level, information relating to severity obtained by the Minnesota code determination, or the like).

The third alert method is to display selected radar chart alone that corresponds to a lead including an abnormal value. More specifically, when there is an abnormal value, the CPU 10 displays a radar chart alone that includes the abnormal value on display 15. In that case, it is desirable that the device displays a heart portion name such as "antero lateral wall" around the radar chart so that users can understand which heart portion is associated with the abnormal value.

The fourth alert method is to utilize audio. More specifically, the device can output an alarm sound when an abnormal value is determined. In alternative embodiments, the device can read a message stating about the presence of abnormal value or read a disease name.

### 8-5. Application embodiments of ECG radar chart device

In the embodiments, the ECG radar chart device 100 is used in emergency medical arenas such as in ambulances or in hospitals. In alternative embodiments, the device can be used in any emergency medical arena in a portable form, used for home medical care by setting the device in a home, or used for living bodies including human or animals.

Devices that have similar functions with that of the ECG radar chart device 100 can be installed in the driver's seat of an automobile or an electric train, an airplane cockpit, or the like, in order to prevent a serious accident from occurring when the driver develops a heart attack due to myocardial infarction etc. In other embodiments, such devices can be installed on a toilet seat, etc., for daily health care. For those applications, it is advantageous for the ECG electrodes 12 to be installed in an area with which the subject's body necessarily makes contact, such as a handle, toilet seat, handrail, or the like.

### 8-6. Embodiments of device configuration

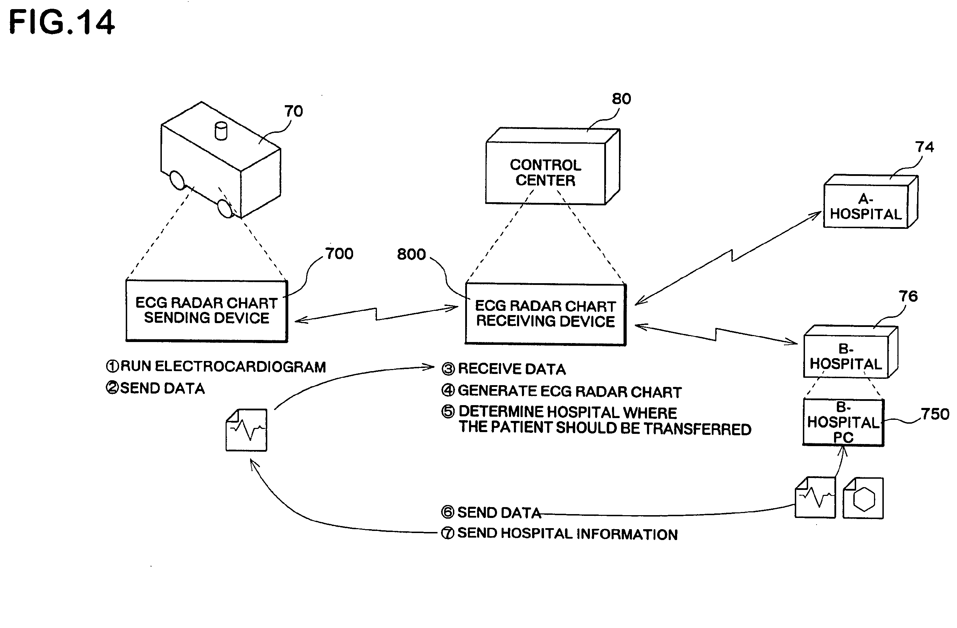

In the embodiments, the ECG radar chart device 100 executes both ECG measurement and ECG radar chart display. In alternative embodiments, those functions can be separately executed by two or more discrete devices. For example, one device can execute an ECG measurement and ECG data output, and the other device can execute an ECG radar chart display based on the ECG data input. An example of a system adopting such a device configuration will be described below together with Fig. 14.

### 8-7. Embodiments of ECG radar chart system

Fig. 14 illustrates the ECG radar chart system as another embodiment of the present invention. The ECG radar chart system includes ambulance 70, in which ECG radar chart sending device 700 is installed, control center 80, in which ECG radar chart receiving device 800 is installed, A-hospital 74, and B-hospital 76, in which a personal computer (which will be described as "B-hospital PC 750") is installed. The system enables the control center to promptly determine which hospital a patient should be transferred to by utilizing an ECG radar chart.

An overview of the system processing will be described. The CPU of the ECG radar chart sending device 700 runs an ECG on a patient (step 1). The CPU sends ECG data and identified value data to the ECG radar chart receiving device 800 (step 2). The CPU of the ECG radar chart receiving device 800 receives the ECG data and identified value data, and stores them (step 3). The CPU of device 800 executes ECG radar chart generating process including analysing process for the data (i.e. feature value analysis and/or disease analysis) and displays an ECG radar chart (step 4). The user (e.g. the doctor) of ECG radar chart receiving device 800 evaluates the patient's heart disease based on the ECG radar chart (refer to Fig. 8), and determines a hospital to which the patient should be transferred (step 5). The CPU of the ECG radar chart receiving device 800 sends ECG data, identified value data, and ECG radar chart data to B-hospital PC 750 at the hospital determined (e.g. B-hospital 76) (step 6). The CPU of the radar chart receiving device 800 sends the name of the hospital determined, the hospital location, etc. to ECG radar chart sending device 700 (step 7).

As mentioned above, in the emergency medical situation, the user can promptly determine to which hospital the patient should be transferred.

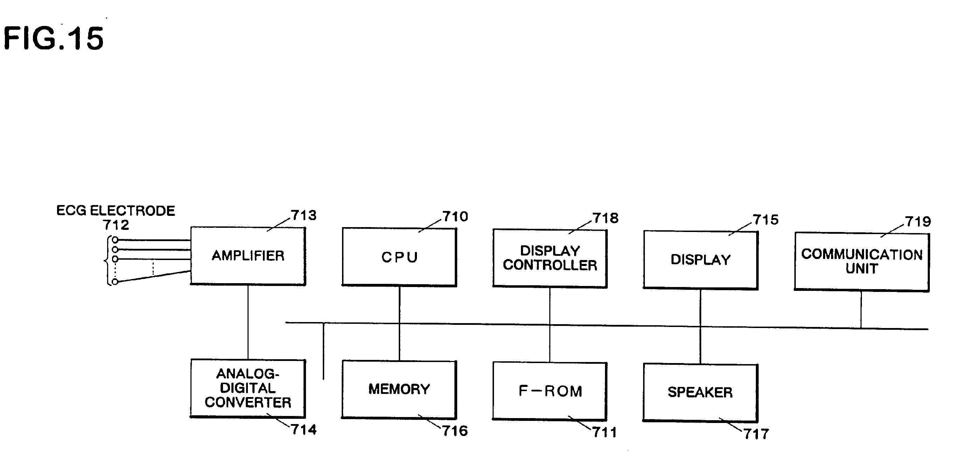

Fig. 15 illustrates a hardware configuration example of the ECG radar chart sending device 700 by use of a CPU. The ECG radar chart sending device 700 includes ECG electrodes 712, amplifier 713, analog-digital converter 714, CPU 710, Flash-ROM 711, memory 716, display controller 718, display 715, speaker 717, and communication unit 719 for communicating with the ECG radar chart receiving device 800 or the like.

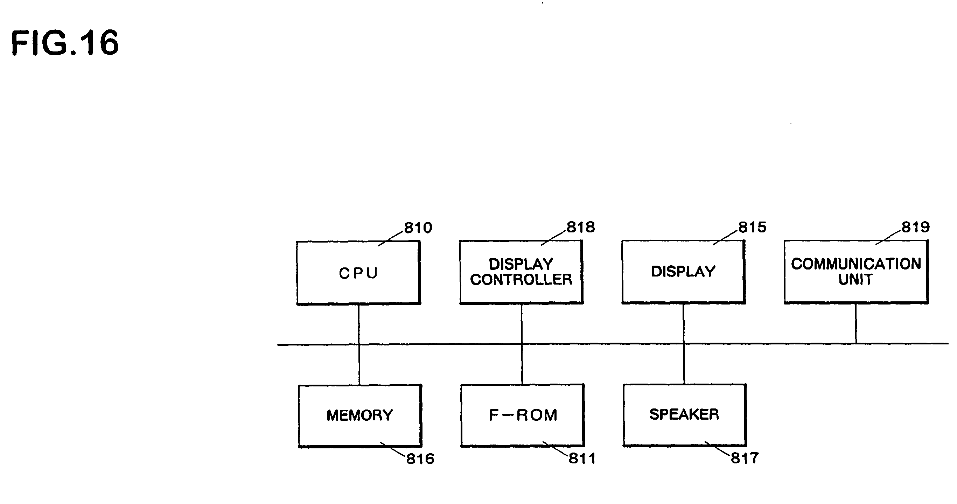

Fig. 16 illustrates a hardware configuration example of the ECG radar chart receiving device 800 by use of a CPU. The ECG radar chart receiving device 800 includes CPU 810, Flash-ROM 811, memory 816, display controller 818, display 815, speaker 817, and communication unit 819 for communicating with the ECG radar chart sending device 700 or the like. A hardware configuration of the B-hospital PC 750 is similar to that of the ECG radar chart receiving device 800. The functions of the hardware component illustrated in Fig. 15 and Fig. 16 are similar to those of the hardware illustrated in Fig. 10.

Communication links between device 700, device 800, and PC 750 can include LAN, EthernetTM, telephone lines, wireless communication, the Internet, wire communication, infrared data communication, mobile phone, Bluetooth, PHS, or the like.

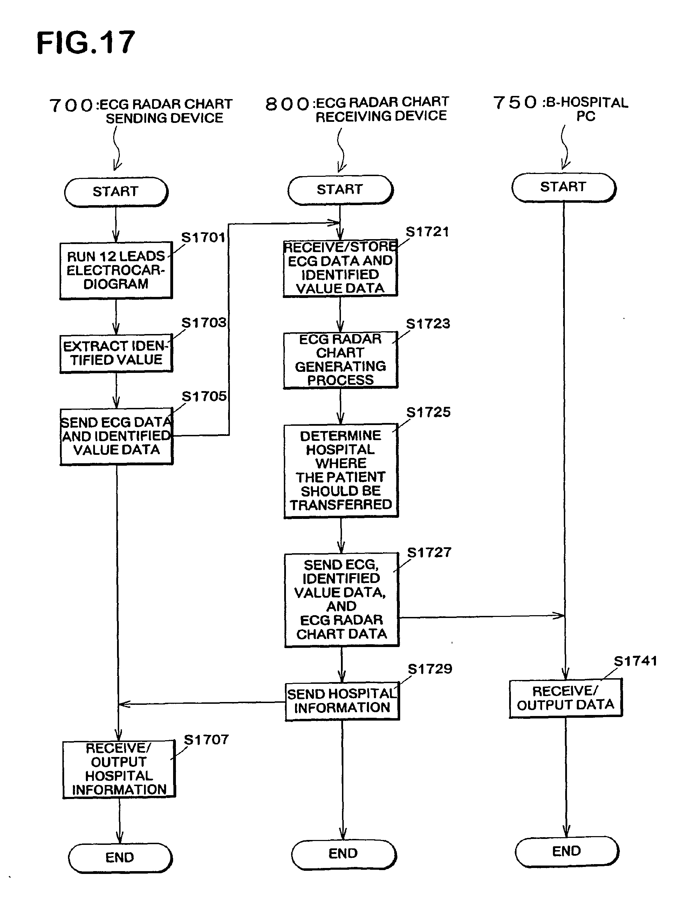

A program flowchart for process of data transmission and reception by the ECG radar chart system will be described below together with Fig. 17.

The CPU 710 of the ECG radar chart sending device 700 runs ECG data from 12 ECG leads, and stores ECG data as results of the ECG measurement in memory 716 (or F-ROM 711) (step 1701 in Fig. 17). The CPU 710 extracts identified values from each of the 12 ECG leads, and stores the identified value data in memory 716 (or F-ROM 711) (step 1703). The CPU 710 sends the ECG data and identified value data (step 1705).

The CPU 810 of the ECG radar chart receiving device 800 receives the ECG data and identified value data, and stores them in memory 816 (or F-ROM 811) (step 1721). The CPU 810 executes the process that generates ECG radar chart and the process including data analyzing process (i.e. feature value analyzing process or disease analyzing process) (step 1723). The ECG radar chart generating process is similar to the processes in step 500 to step 421 in Fig. 4.

The user (e.g. the doctor) of the ECG radar chart receiving device 800 checks whether the ECG radar chart displayed as the processing result of step 1723 shows an abnormal value or not (refer to Fig. 8). The user evaluates the patient's heart disease, and determines to which hospital the patient should be transferred, based on the ECG radar chart. The user inputs the determination results in the device 800. The CPU 810 obtains the determination information etc. of the hospital (step 1725).

The CPU 810 sends the ECG data, the identified value data, and ECG radar chart data (step 1727). The CPU of B-hospital PC 750 receives those data, and outputs them to a display etc. (step 1741). The CPU 810 sends the determination information about the hospital, etc., to the ECG radar chart sending device 700 (step 1729). The CPU 710 of ECG radar chart sending device 700 receives the determination information about the hospital etc., and outputs them to a display 715, etc. (step 1707).

As mentioned above, the ECG radar chart receiving device 800 receives the ECG data and identified value data. In an alternative embodiment, the device receives data that is to display radar charts based on the ECG data (which corresponds to ECG radar chart data or chart data that is to display a radar chart that relates the feature value to each portion of the heart). In communications between the ECG radar chart sending device 700 and the ECG radar chart receiving device 800, the ECG data and/or identified value data as well as the ECG radar chart data can be subjects for data transmission and reception. In communications between the ECG radar chart receiving device 800 and B-hospital PC 750, subjects for data transmission and reception can be any of the ECG data, identified value data, or ECG radar chart data.

### 8-8. Program execution

In the embodiments, the computer program for the CPU 10 is stored in the F-ROM 11. The computer program can be installed on the hard disk etc. from an installation CD-ROM (not shown). In alternative embodiments, the program can be installed from computer-readable storage media such a flexible disk (FD) or IC card (not shown). Alternatively, the program can be downloaded to the devices via the communications lines. The program can also be installed on the devices from the CD-ROM, and the device executes the installed program. In alternative embodiments, the device can directly execute the program stored on the CD-ROM.

Computer-executable programs used in the embodiments include a program to be executable just after installation, a program that needs to be converted to another format (e.g. decompressing compressed data), or a program to be executable within a module.

A general description of the present invention as well as preferred embodiments of the invention has been set forth above. It is to be expressly understood, however, the terms described above are for purpose of illustration only and are not intended as definitions of the limits of the invention. Those skilled in the art to which the present invention pertains will recognize and be able to practice other variations in the system, device, and methods described which fall within the teachings of this invention. Accordingly, all such modifications are deemed to be within the scope of the invention.

## Claims

1. An electrocardiogram (ECG) analysis device for analyzing an ECG, comprising:
means for analyzing level of an ECG feature value;
means for determining disease information relating to a patient's disease based on information including the feature value; and
means for outputting both the feature value analysis result analyzed
by the ECG feature value analyzing means and the disease information result determined by the disease information determining means.

2. A computer program for an ECG analysis device that analyzes an ECG, wherein the program is implemented in a computer and capable of causing the computer to perform:
means for analyzing magnitude of an ECG feature value;
means for determining disease information relating to a patient's disease based on information including the feature value; and
means for outputting both the feature value analysis result analyzed
by the ECG feature value level analyzing means and the disease information result determined by the disease information determining means.

3. The ECG analysis device or the computer program according to claims 1 or 2, wherein the outputting means further displays a chart that relates the feature value analysis result to each portion of heart.

4. The ECG analysis device or the computer program according to claims 3, wherein the outputting means further displays the chart in a radar chart form that arranges each of the feature value analysis result at the corresponding portion of the heart.

5. The ECG analysis device or the computer program according to claims 3 or 4, wherein the outputting means further outputs history of the feature value analysis result and/or history of the disease information result when outputting the feature value analysis result.

6. The ECG analysis device or the computer program according to claims 3, 4, or 5, wherein the outputting means further outputs history summary of the feature value analysis result.

7. The ECG analysis device or the computer program according to one of claims 1-6, wherein the feature value is based on the constituent elements of an ECG including P wave, Q wave, R wave, S wave, ST segment, or T wave.

8. The ECG analysis device or the computer program according to one of claims 1-7, wherein the disease information determining means determines the disease information based on the Minnesota code as an ECG classification reference.

9. The ECG analysis device or the computer program according to one of claims 1-8, wherein the ECG analysis device further outputting heartbeat-related information by sound and/or varying display style during analyzing the ECG.

10. The ECG analysis device or the computer program according to one of claims 1-9, wherein the ECG analysis device further outputting a warning signal when the analysis can not be executed during analyzing the ECG.

11. An ECG analysis device for analyzing an ECG, a central processing unit (CPU) of the ECG analysis device is to execute the procedures of:
analyzing level of an ECG feature value;
determining disease information relating to a patient's disease based on information including the feature value; and
outputting both the feature value analysis result and the disease information result.

12. A method for analyzing an ECG comprising the steps of:
analyzing magnitude of an ECG feature value;
determining disease information relating to a patient's disease based on information including the feature value; and
outputting both the feature value analysis result and the disease information result.

13. A method for analyzing an ECG comprising the steps of:
analyzing magnitude of an ECG feature value;
determining disease information relating to a patient's disease based on information including the feature value;
narrowing down the candidates of disease information result based on the feature value analysis result; and
outputting the narrowed disease information result candidates.

14. A method for analyzing an ECG comprising the steps of:
analyzing magnitude of an ECG feature value;
determining disease information relating to a patient's disease based on information including the feature value;
determining different disease information than the determined disease information by considering both the feature value analysis result and the determined disease information result; and
outputting the different disease information result.

15. A method for analyzing an ECG comprising the step of analyzing the ECG by combining an algorithm for analyzing level of an ECG feature value and an algorithm for determining whether a patient's cardiac function is abnormal or not, which is based on information including the feature value.
